# EUROPEAN PATENT APPLICATION

(11) **EP 3 632 473 A2**
(43) Date of publication of application: **08.04.2020**
(21) Application number: 19197965.7
(22) Date of filing: 18.09.2019
(51) Int. Cl.: A61L 2/025, A23G 9/30

(54) **PROCESS AND MACHINE FOR THE ULTRASONIC TREATMENT OF LIQUID FOOD MIXTURES**

(30) Priority: 05.10.2018 IT 201800009191
(71) Applicant: BRAVO S.p.A., 36075 Montecchio Maggiore (Vicenza) (IT)
(72) Inventor: Bravo, Stefano, 36050 SOVIZZO (VI) (IT); Bravo, Giuseppe, 36050 SOVIZZO (VI) (IT)
(74) Representative: Martegani, Franco

(57) **Abstract**

A process for the ultrasonic treatment of a liquid food mixture, in particular for the preparation of ice-cream, pastry and gastronomy products. According to the invention the mixture is contained inside a container which is penetrated directly by said ultrasounds, without the interpositioning of any additional transmission element of the same ultrasounds to the mixture.

## Description

The present invention relates to a process for the ultrasonic treatment of liquid food mixtures contained within a container, in particular mixtures for the preparation of ice-cream, pastry and gastronomy products.

The invention also relates to an example of a machine for implementing the process.

International patent application WO2017/216703 A1 describes a process and an apparatus/device for maturing food through the use of ultrasounds.

The food, in particular meat, is immersed in a liquid contained within a casing of plastic material impermeable to the liquid but permeable to ultrasonic waves.

The casing of plastic material containing the food to be treated is in turn immersed in a liquid contained in a vacuum-packed tank; said liquid is heated and recirculated by a pump.

More details can be found from examining WO2017/216703A1.

This process of the prior art, however, is not suitable for the treatment of liquid food mixtures.

The general objective of the present invention is to provide a process and a machine capable of treating, by means of ultrasounds, liquid food mixtures, in particular mixtures for the preparation of ice-cream, pastry and gastronomy products.

This objective is achieved by a process and a machine produced according to claim 1 and the enclosed subordinate claims.

More specifically, the invention relates to a process for the ultrasonic treatment of a liquid food mixture, in particular for the preparation of ice-cream, pastry and gastronomy products, characterized in that said mixture is contained within a container which is directly penetrated by said ultrasounds so that it becomes a sounding board, without the interpositioning of any additional transmission element of the same ultrasounds to the mixture.

The invention also relates to a machine for implementing the process characterized in that it comprises in combination a container/tank suitable for containing the mixture to be treated, an ultrasonic emitter operatively connected to said container/tank, a control unit of said emitter and an electronic control card of said control unit according to the type of mixture to be treated.

The characteristics of the invention and its advantages with respect to the known art will be even more clearly understandable from the following description, referring to the attached drawings, in which:
- figure 1 is a perspective view partially illustrating an example of a machine for implementing the process according to the invention;
- figure 2 is a plan view of the machine of figure 1;
- figure 3 is a block diagram illustrating a control system of the machine of figures 1 and 2.

Ultrasounds are mechanical sound waves. Unlike so-called acoustic phenomena, the frequencies that characterize ultrasounds are higher than those normally heard by a human ear. The frequency conventionally used for discriminating sonic waves from ultrasonic waves is set at 20 kHz.

Ultrasounds are generated by means of materials with particular mechanical-electrical characteristics, piezoelectric materials.

A second system for generating ultrasounds is based on magnetostriction: a ferromagnetic core subjected to an alternating magnetic field (maximum 200 kHz) is vibrated at ultrasonic frequencies. This system is used, for example, for the production of industrial ultrasonic washing machines.

Food subjected to ultrasounds undergoes a treatment that may have various positive characteristics - a sterilizing effect, ultrasounds, in fact, reduce the bacterial charge of food at very low temperatures, preserving the organoleptic quality.

A homogenizing effect of the components of the mixtures treated has also been found.

Sonication in the ice-cream mixture increases the nucleation of ice crystals and therefore allows faster freezing and greater control over the size of the crystals themselves. All of this produces a softer ice-cream structure, as cavitation allows an optimization of the ice phase. As a whole, high intensity and low frequency is a technique that favours the homogenization and, consequently, the mixing of the ingredients in a more uniform structure.

Sonication not only leads to the production of air cells in ice-cream, but, through "ice nucleation", it accelerates the freezing process, preventing the formation of encrustation on the surface of the freezer and removing solid ice on the same.

Thanks to these phenomena, the freezing time is generally reduced by up to 30%, thus also obtaining a reduction in energy. Ultrasound treatment takes place after homogenization and maturation in industrial ice-cream. In artisan ice-cream, at the end of the process on the mixture already treated and before starting the "freezing".

The essential parameters of the system are intensity, energy and amplitude, in addition to the treatment time.

A homogenizing effect of the components of the mixtures treated has also been found. Homogenization is extremely important for ice-cream and pastry mixtures as it makes the final product more homogeneous and durable over time as it does not dissociate. Conventional homogenization treatment is extremely stressful, however, as it passes the mixture through a very high pressure diaphragm which also overheats it. The advantage of these machines is that they can handle huge quantities of product continuously. Ultrasounds can treat mixtures similarly to homogenization but in a compact way. In parallel with the homogenizing effect, there is also the thickening and emulsifying effect. All this because the mechanical waves act as a high-energy molecular mixer.

In chocolate there is an adjuvant effect of nucleating the β(V) crystals, at the corresponding temperature, which helps to obtain a good number of final crystals, the main purpose of tempering.

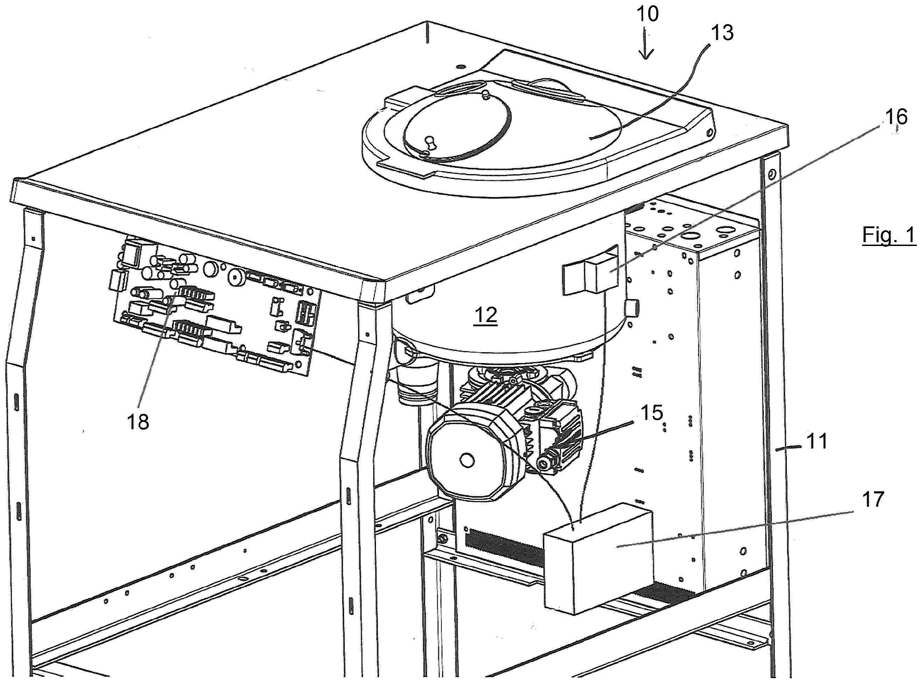

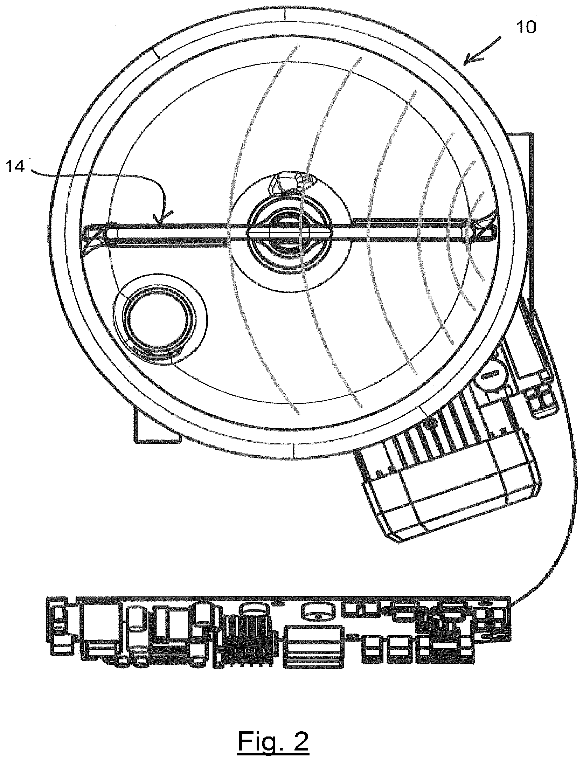

With reference to figures 1 and 2 of the drawings, an example of a machine for implementing the process according to the invention is indicated as a whole with 10.

Said machine is structurally composed of a framework 11 for supporting and containing a container/tank 12 with an opening/closing lid 13.

A mixer 14 is mounted inside the container 12, caused to rotate, at different speeds according to the type and consistency of the mixture to be treated, for example by means of a gearmotor group 15.

The container/tank 12 can be of any material suitable for the purpose, for example stainless steel or food-grade plastic material.

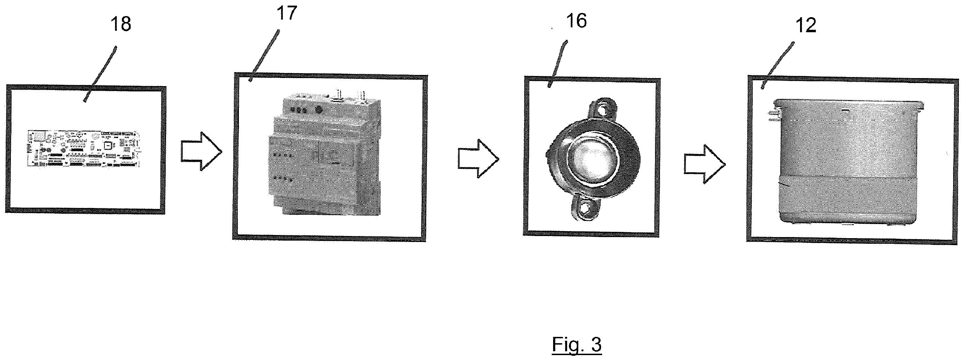

An ultrasound emitter group 16 is applied to the outer casing of the container/tank 12, controlled by a control unit 17 which is controlled by an electronic card 18 (figures 2 and 3). The control of the control unit 17 is automatic through the implementation of programs on the electronic card 18.

Means for sealing the container/tank 12 can also be associated with the lid 13.

To supply the machine with this technology, a device must be applied that emits ultrasounds at the edge of the tank. Depending on the type of tank, the material with which it is made, the geometry and the intended purpose, the most suitable emitter must be identified and the frequency tuned so that the maximum efficiency of the process can be obtained.

This emitter is controlled by a control unit that produces the desired frequencies and also manages the power emitted.

The control unit is controlled by the electronic card which, based on the recipe started, requires the intervention of the control unit in the ways specified in the recipe in a completely automatic way.

The tank to which this device is applied can be equipped with heating, cooling/freezing devices, mixers/ stirrers. It is a tank closed by a lid accessible by the user, who can insert products that complete the recipe.

The objective mentioned in the preamble of the description has therefore been achieved.

The scope of the invention is defined by the following claims.

## Claims

1. A process for the ultrasonic treatment of a liquid food mixture, in particular for the preparation of ice-cream, pastry and gastronomy products, **characterized in that** said mixture is contained inside a container that is penetrated directly by said ultrasounds, without the interpositioning of any additional transmission element of the same ultrasounds to the mixture.

2. The process according to claim 1, **characterized in that** said container is made of metal.

3. The process according to claim 1, **characterized in that** said container is made of plastic material.

4. The process according to claim 1, **characterized in that** the power of said ultrasounds is regulated according to the type of mixture to be treated.

5. A machine for implementing the process according to any of the claims from 1 to 4 **characterized in that** it comprises, in combination: a container/tank (12) suitable for containing the mixture to be treated, an ultrasound emitter (16) operatively connected to said container/tank (12), a control unit (17) for controlling said emitter (16), and an electronic card (18) for controlling said control unit (17) according to the type of mixture to be treated.

6. The process according to claim 5, **characterized in that** the control of the control unit (17) is automatic through the implementation of programs of the electronic card (18).

7. The process according to claim 5, **characterized in that** said emitter (16) is applied on the outer casing of said container/tank (12).

8. The process according to claim 5, **characterized in that** said emitter (16) is inside the tank, immersed in the mixture to be treated.
